# EUROPÄISCHE PATENTSCHRIFT

(11) **EP 1 320 727 B1**
(45) Veröffentlichungstag und Bekanntmachung des Hinweises auf die Patenterteilung: **10.08.2005**
(21) Anmeldenummer: 00927106.5
(22) Anmeldetag: 28.04.2000
(51) Int. Cl.: G01F 23/26, G01F 11/28

(54) **FLÜSSIGKEITSRESERVOIR MIT FÜLLSTANDSMESSUNG UND DOSIERSYSTEM, ENTNAHMESYSTEM SOWIE KOMBINIERTES DOSIER/ENTNAHMESYSTEM**
FLUID RESERVOIR WITH LEVEL MEASUREMENT AND A DOSING SYSTEM, A WITHDRAWAL SYSTEM AND A COMBINED DOSING/WITHDRAWAL SYSTEM
RESERVOIR DE LIQUIDE AVEC MESURE DU NIVEAU ET SYSTEME DOSEUR ET SYSTEME COMBINE DE DOSAGE/SOUTIRAGE

(43) Veröffentlichungstag der Anmeldung: 25.06.2003
(73) Patentinhaber: Fraunhofer-Gesellschaft zur Förderung der angewandten Forschung e.V., 80686 München (DE)
(72) Erfinder: RICHTER, Martin, D-81677 München (DE); WOIAS, Peter, D-79102 Freiburg (DE)
(74) Vertreter: Schoppe, Fritz, Dipl.-Ing.
(86) Internationale Anmeldenummer: PCT/EP2000/003878
(87) Internationale Veröffentlichungsnummer: WO 2001/084091

(56) Entgegenhaltungen:
- EP-A- 0 458 405
- DE-A- 4 306 061
- US-A- 5 224 375
- US-A- 5 463 228
- PATENT ABSTRACTS OF JAPAN vol. 013, no. 242 (P-880), 7. Juni 1989 (1989-06-07) & JP 01 044852 A (TAKESHI TAKAOKI), 17. Februar 1989 (1989-02-17)

## Beschreibung

Die vorliegende Erfindung bezieht sich auf ein Flüssigkeitsreservoir und insbesondere auf ein Flüssigkeitsreservoir mit Füllstandsmessung, das in Verbindung mit einem Dosiersystem, einem Entnahmesystem oder einem Dosier/Entnahmesystem verwendet werden kann.

Auf dem medizinischen Sektor, aber auch auf sonstigen Gebieten der Technik besteht der Bedarf, Flüssigkeitsmengen zu bestimmen, um eine absolute Flüssigkeitsmessung durchzuführen, oder aber um bestimmte Flüssigkeitsmengen zu dosieren. zur Bestimmung der Menge der abgegebenen Flüssigkeit ist dann nicht mehr die Flüssigkeitsmenge an sich, sondern die Änderung der Flüssigkeitsmenge mit der Zeit maßgeblich.

Insbesondere auf dem medizinischen Sektor besteht auch ein großer Bedarf nach Flüssigkeitsreservoiren für Medikamente. Hier müssen oftmals genau dosierte Flüssigkeitsmengen verabreicht werden. Darüber hinaus besteht der Bedarf, daß die Flüssigkeitsreservoire preisgünstig ausgeführt sind, da es sich oftmals um Einwegartikel handelt, welche aus hygienischen Gründen nicht wieder verwendet werden können.

Das U.S.-Patent Nr. 5,463,228 offenbart eine Vorrichtung zum automatischen exakten Dosieren von kleinen Flüssigkeitsmengen in einem medizinischen Analysesystem, wobei die Vorrichtung eine transparente Meßröhre mit einer Kapillarröhre aufweist, die einen Innendurchmesser von weniger als 1 mm hat, wobei die Meßröhre ferner eine Flüssigkeitsiibertragungsöffnung an einem ersten Ende derselben aufweist, wobei die Flüssigkeitsübertragungsöffnung für ein Ansaugen einer Flüssigkeit vorgesehen ist. Die Vorrichtung umfaßt ferner eine Fluidphasengrenzerfassungseinrichtung zum automatischen Erfassen einer Fluidphasengrenze in der Meßröhre, wobei ein elektrisches Positionssignal einer Position der Fluidphasengrenze erzeugt wird. Die Erfassung der Fluidphasengrenze findet optisch statt, und zwar unter Verwendung einer Lichtquelle und einer CCD-Zeile.

Die DE 4306061 Al offenbart eine Vorrichtung zur Detektion des Füllstandes eines kapillaren Überlaufkanals. Die Vorrichtung umfaßt einen Kanal, der an einem Ende mit einem Zulauf verbunden ist, das über ein steuerbares Ventil mit einem vorratsbehälter verbunden ist. Am anderen Ende des Kanals befindet sich ein weiterer Kanal größeren Durchmessers, der mit einem Ablauf verbunden ist. Fluidmäßig mit dem Kanal verbunden ist ein Überlaufkanal, der schraubenförmig um den Kanal herum ausgebildet ist, und der sich zwischen einer ersten Erfassungselektrode und einer zweiten Erfassungselektrode erstreckt, um den Füllstand des Überlaufkanals kapazitiv zu erfassen. Der Überlaufkanal wirkt als Puffervolumen. Wird ein Füllstand im Überlaufkanal detektiert, so wird das Ventil zwischen dem Vorratsbehälter und dem Zulauf geschlossen, bis detektiert wird, daß der Überlaufkanal leer ist. Dann wird das Ventil wieder geöffnet, bis der Überlaufkanal wieder einen Füllstand hat, worauf das Ventil wieder geschlossen wird. Die Regelvorrichtung kann zur Trennung eines Tuschevorratsbehälters bei Plotterstiften, Registriergeräten, Medizingeräten oder Geräten in der Prozeßtechnik zur Anwendung kommen.

Die Aufgabe der vorliegenden Erfindung besteht darin, ein Dosiersystem oder ein kombiniertes Dosier/Entnahmesystem mit einem Flüssigkeitsreservoir zu schaffen.

Diese Aufgabe wird durch ein Dosiersystem nach Patentanspruch 1 oder ein kombiniertes Dosier/Entnahmesystem nach Anspruch 2 gelöst.

Der vorliegenden Erfindung liegt die Erkenntnis zugrunde, daß eine genaue Füllstandsmessung einer Flüssigkeit in einem Flüssigkeitsreservoir dann erreicht werden kann, wenn das Flüssigkeitsreservoir einen in einem Basiskörper ausgebildeten Kanal mit einem Einlaß und einem Auslaß aufweist, wobei die Querschnitts-Dimensionierung des Kanals so gewählt ist, daß eine in den Kanal füllbare Flüssigkeit einen Flüssigkeitsmeniskus bildet, der einen mit Flüssigkeit gefüllten Abschnitt des Kanals von einem ungefüllten Abschnitt des Kanals, d.h. einem Abschnitt des Kanals, in dem keine Flüssigkeit vorhanden ist, abgrenzt, wobei die Position des Meniskus bezüglich des Kanals von der Ausrichtung des Flüssigkeitsreservoirs im wesentlichen unabhängig ist. Zur Füllstandsmessung wird eine Detektionseinrichtung zum Erfassen der Position des Flüssigkeitsmeniskus bezüglich des Kanals eingesetzt, um aus der Position des Flüssigkeitsmeniskus den Füllstand und/oder eine Änderung des Füllstands des Flüssigkeitsreservoirs zu erhalten.

Allgemein gesagt hängen Position und Form des Flüssigkeitsmeniskus von der Schwerkraft einerseits sowie der Oberflächenspannung zwischen Flüssigkeit und Luft und der Grenzflächenspannung zwischen Medikament und Reservoirmaterial andererseits ab. Die beiden letzten Parameter definieren den Benetzungswinkel. Erfindungsgemäß wird die Querschnitt-Dimensionierung des Kanals so klein gewählt, daß die Form des Flüssigkeitsmeniskus vor allem durch die Oberflächen- und Grenzflächenspannungen und nicht mehr durch die Schwerkraft oder andere Kräfte, z. B. Rotationskraft, Vibrationskraft, magnetische Kräfte, etc., bestimmt wird. Je nach Flüssigkeitseigenschaften und Kanalmaterial wird bei im Querschnitt kreisförmigen Kanälen bei Durchmessern kleiner als 0,5 bis 3 mm die Oberflächenspannung (und auch die Grenzflächenspannung) gegenüber der Schwerkraft dominieren. Dann spielt die Schwerkraft keine Rolle mehr, d.h. auch bei einer beliebigen Position des Flüssigkeitsreservoirs, sei es, daß dasselbe gekippt ist, auf dem Kopf stehend ist oder irgendwie angeordnet ist, wird der Flüssigkeitsmeniskus seine Position nicht signifikant ändern.

Je nach Ausführungsform können eine kapazitive Detektion der Position des Füllstandsmeniskus bezüglich des Kanals oder auch eine optische Detektion sowie andere Detektionsmittel eingesetzt werden. Sämtliche Detektionsmittel basieren auf unterschiedlichen Eigenschaften des ungefüllten Abschnitts des Kanals im Vergleich zum gefüllten Abschnitt des Kanals.

Die vorliegende Erfindung ist besonders darin vorteilhaft, daß sie sehr preisgünstig implementiert werden kann, insbesondere wenn kapazitive Detektionsverfahren eingesetzt werden, da dann lediglich zwei Elektroden bezüglich des Kanals angebracht werden müssen, durch die ein elektrisches Feld erzeugbar ist, das sich sowohl im ungefüllten Abschnitt, als auch im gefüllten Abschnitt des Kanals erstreckt. Dieser Vorteil der Preisgünstigkeit ist besonders auf dem sehr wettbewerbsintensiven Massenmarkt der Einweg-Produkte im medizinischen Bereich erheblich.

Das Dosiersystem oder das kombinierte Dosier/Entnahmesystem der vorliegenden Erfindung umfaßt ein Flüssigkeitsreservoir dessen Füllstand völlig unabhängig von der Position und Lage des Flüssigkeitsreservoirs genauer bestimmt werden kann. Dies ist wiederum bei Flüssigkeitsreservoiren zur Aufnahme von Medikamenten von großer Bedeutung, da insbesondere bei einem Einsatz solcher Flüssigkeitsreservoire zur dauernden Dosierung von Medikamenten solche Flüssigkeitsreservoire von Patienten getragen werden und daher ständig ihre Position und Ausrichtung verändern. Aufgrund der erfindungsgemäßen Dimensionierung des Kanals bleibt jedoch der Flüssigkeitsmeniskus immer an derselben Position, da seine Position nicht mehr von der Schwerkraft abhängt, sondern lediglich von der Oberflächenspannung des Medikaments und der Grenzflächenspannung zwischen Medikament und Kanalwand.

Ein weiterer Vorteil der vorliegenden Erfindung besteht darin, daß das Behältervolumen des Flüssigkeitsreservoirs mit erfindungsgemäßem Kanal dennoch beträchtliche Werte annehmen kann. Dies kann einerseits dadurch erreicht werden, daß der Kanal mäanderförmig in dem Basiskörper angeordnet wird, so daß sich eine maximale Kanallänge im Vergleich zu den äußeren Abmessungen des Basiskörpers ergibt. Alternativ ist es auch grundsätzlich möglich, wenn Platzbedürfnisse für den Behälter nicht entscheidend sind, das Volumen sehr groß zu gestalten, da die Bildung eines Meniskus, dessen Position im Kanal von der Schwerkraft im wesentlichen unabhängig ist, nicht von der Querschnittsfläche des Kanals abhängt, sondern von der Form des Kanalquerschnitts, genauer gesagt der kleinsten Dimension des Kanalquerschnitts. wird ein rechteckiger Kanal betrachtet, so bildet sich immer dann ein Meniskus, wenn eine Seitenlänge des Kanalquerschnitts so klein dimensioniert ist, daß die Oberflächenspannung der Flüssigkeit zu einer Meniskusbildung führt. Die andere Seitenlänge des Kanalquerschnitts kann dagegen prinzipiell beliebig große Werte annehmen, so daß das Behältervolumen des Flüssigkeitsreservoirs in großen Grenzen eingestellt werden kann. Für praktische Anwendungen insbesondere im Bereich der Medikamentendosierung sind jedoch Volumen im Bereich von 0,1 bis 50 ml ausreichend, so daß die erfindungsgemäßen Flüssigkeitsreservoire noch handlich sind.

Ein weiterer Vorteil der vorliegenden Erfindung besteht darin, daß, wenn der Basiskörper als Schlauch ausgeführt ist, ohne weiteres ein handelsüblicher Schlauch mit entsprechend geringem Querschnittsdurchmesser als Flüssigkeitsreservoir eingesetzt werden kann, da er lediglich um z. B. eine kapazitive Detektionseinrichtung ergänzt werden muß, um ein preisgünstiges, flexibles und genaues Flüssigkeitsreservoir zu erhalten.

Es sei darauf hingewiesen, daß die Querschnitts-Dimensionierung des Kanals über der Länge des Kanals nicht unbedingt konstant sein muß. Eventuelle Querschnittserhöhungen oder -verringerungen können ohne weiteres herauskalibriert werden bzw. bei der Bestimmung des Füllstandsvolumens über einen von der Meniskusposition abhängigen Umrechnungsfaktor berücksichtigt werden.

Ein weiterer Vorteil der vorliegenden Erfindung besteht darin, daß sich das Meniskuskonzept aufgrund der relativ kleinen Kanalabmessungen nahezu optimal mit einem kapazitiven Meßprinzip ergänzt. Allgemein ist eine gemessene Kapazität umso größer, je kleiner der Abstand zwischen den Kapazitätselektroden ist. So ist auch der Meniskus umso stabiler, je kleiner die Abmessungen des Kanals sind. Für das kapazitive Meßprinzip bedeutet dies jedoch, daß abhängig von der Geometrie und den dielektrischen Parametern der Flüssigkeit und des Reservoirmaterials eine ausreichend große Empfindlichkeit in Form einer ausreichend großen Kapazitätsänderung bei der Bewegung des Meniskus, die durch eine Abgabe der Flüssigkeit aus dem Flüssigkeitsreservoir bedingt wird, auftritt.

Ein weiterer Vorteil der vorliegenden Erfindung besteht darin, daß durch preisgünstige aber effiziente Konzepte die Verdunstung der Flüssigkeit in dem Flüssigkeitsreservoir je nach Bedürfnissen reduziert bzw. voll und ganz ausgeschaltet werden kann, ohne daß aufwendige Maßnahmen erforderlich sind.

Bevorzugte Ausführungsbeispiele der vorliegenden Erfindung werden nachfolgend bezugnehmend auf die beiliegenden Zeichnungen detailliert erläutert. Es zeigen:
- Fig. 1: eine detaillierte Darstellung eines erfindungsgemäßen Flüssigkeitsreservoirs mit Füllstandsmessung, das eine kapazitive Detektion verwendet.
- Fig. 2: eine graphische Darstellung des Kapazitätsverlaufs über der Meniskusposition für das erfindungsgemäße Flüssigkeitsreservoir von Fig. 1;
- Fig. 3: eine Draufsicht auf einen Basiskörper eines erfindungsgemäßen Flüssigkeitsreservoirs, das ebenfalls eine kapazitive Erfassung der Position des Meniskus verwendet, bei dem jedoch Vertikalelektroden vorhanden sind;
- Fig. 4: eine Draufsicht auf die Bodenelektrode und die Deckelelektrode, welche bei dem in Fig. 4 gezeigten Ausführungsbeispiel jeweils aus einer Mehrzahl von Einzelelektroden bestehen;
- Fig. 5: eine perspektivische Ansicht eines erfindungsgemäßen Flüssigkeitsreservoirs mit Füllstandsmessung, bei dem der Basiskörper als Schlauch ausgeführt ist; und
- Fig. 6: eine perspektivische Ansicht eines erfindungsgemäßen Flüssigkeitsreservoirs mit Füllstandsmessung, bei dem ebenfalls wie in Fig. 5 der Basiskörper als Schlauch ausgeführt ist, bei dem jedoch eine koaxiale Anordnung der beiden Elektroden verwendet wird.

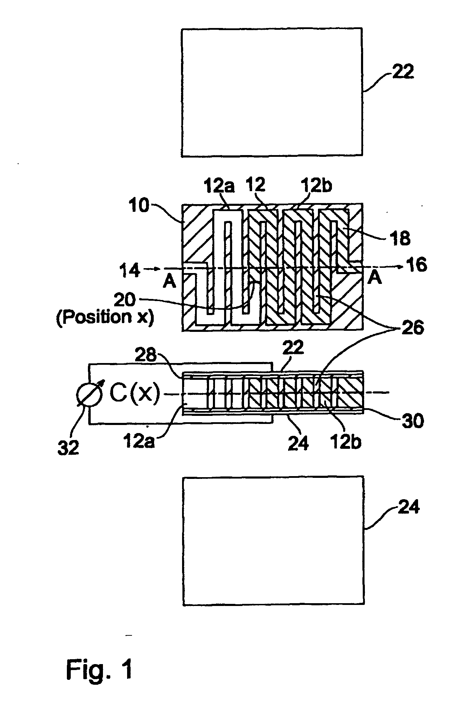

Fig. 1 zeigt ein erfindungsgemäßes Flüssigkeitsreservoir mit Füllstandsmessung, bei dem das bevorzugte kapazitive Erfassungsprinzip verwendet wird. Das erfindungsgemäße Flüssigkeitsreservoir umfaßt einen Basiskörper 10, in dem eine vorzugsweise mäanderförmiger Kanal 12 gebildet ist, der einen Einlaß 14 und einen Auslaß 16 aufweist. Die Querschnitts-Dimensionierung des Kanals ist so gewählt, daß eine in den Kanal füllbare Flüssigkeit 18 einen Flüssigkeitsmeniskus 20 bildet, der den Kanal in einen ungefüllten Abschnitt 12a und in einen in Fig. 1 schraffiert gezeigten gefüllten Abschnitt 12b aufteilt. Der ungefüllte Abschnitt 12a des Kanals erstreckt sich vom Einlaß 14 bis zum Flüssigkeitsmeniskus 20, während sich der gefüllte Abschnitt 12b des Kanals vom Flüssigkeitsmeniskus 20 bis zum Auslaß 16 erstreckt. In dem ungefüllten Abschnitt 12a des Kanals befindet sich vorzugsweise Luft. Dieser Abschnitt ist üblicherweise auf Umgebungsdruck. Der gefüllte Abschnitt 12b ist abgesehen von eventuell vorhandenen unerwünschten Gasblasen vollständig mit der Flüssigkeit 18 gefüllt.

Wenn das erfindungsgemäße Flüssigkeitsreservoir gemäß seiner bevorzugten Anwendung als Medikamentenreservoir eingesetzt wird, so ist die Flüssigkeit 18 irgendein Medikament in flüssiger Phase. Alternativ kann das erfindungsgemäße Flüssigkeitsreservoir auch zur Aufnahme anderer Flüssigkeiten verwendet werden.

In einer bevorzugten Ausführungsform der vorliegenden Erfindung, wie es in Fig. 1 gezeigt ist, wird ein kapazitives Erfassungsprinzip verwendet. Hierzu sind zwei Elektroden 22 und 24 so auf dem Basiskörper aufgebracht, daß sich ein zwischen den zwei elektrisch isolierten Elektroden erzeugbares elektrisches Feld sowohl in dem gefüllten Abschnitt 12b als auch in dem ungefüllten Abschnitt 12a erstreckt, so daß eine Positionsänderung des Flüssigkeitsmeniskus 20 aufgrund der unterschiedlichen Dielektrizitätszahlen des Medikaments 18 und der Luft im ungefüllten Abschnitt 12a zu einer proportionalen Kapazitätsänderung führt. Je nach Ausgestaltung der Elektroden, des Kanalquerschnitts und der Gestaltung des Kanals kann ein linearer Zusammenhang zwischen der Kapazitätsänderung und der Wegänderung des Flüssigkeitsmeniskus 20 erreicht werden.

Im zweituntersten Teilbild von Fig. 1 ist ein Querschnitt durch das erfindungsgemäße Flüssigkeitsreservoir entlang der Linie AA des zweitobersten Teilbilds von Fig. 1 gezeigt. Das erfindungsgemäße Flüssigkeitsreservoir umfaßt die eine Elektrode 22, die auch aufgrund der in Fig. 1 gezeigten Darstellung als Deckelelektrode bezeichnet wird, sowie die andere Elektrode 24, die auch als Bodenelektrode bezeichnet wird. Darüber hinaus ist der Kanal durch seinen ungefüllten Abschnitt 12a und durch seinen schraffiert gezeichneten gefüllten Abschnitt 12b dargestellt. Die Mäandernatur des Kanals 12 äußert sich durch Stege 26 des Basiskörpers, die die einzelnen Kanalabschnitte voneinander trennen. Wie es in Fig. 1 gezeigt ist, sind die Elektroden 22, 24 jeweils durch Isolationsschichten 28, 30 von dem Kanal isoliert. Dies ist dann erforderlich, wenn die Flüssigkeit 18 elektrisch leitfähig ist. Ist die Flüssigkeit 18 jedoch ohnehin elektrisch isolierend, so kann auch auf die Isolationsschichten 28, 30 verzichtet werden, und die Bodenelektrode 24 und die Deckelelektrode 22 können direkt den Kanal 12 nach oben und nach unten begrenzen. Hierbei sind jedoch im Falle eines Medikamentenreservoirs auch medizinische Gesichtspunkte zu berücksichtigen hinsichtlich Materialvorschriften, Materialauswahl oder Medikamentsubstanz, d. h. ob dieselbe direkt mit unter Spannung stehenden Elektroden in Verbindung gebracht werden darf.

Das erfindungsgemäße Flüssigkeitsreservoir umfaßt schließlich eine Detektionseinrichtung 32, die bei dem in Fig. 1 gezeigten bevorzugten Ausführungsbeispiel der vorliegenden Erfindung eine Kapazitätsmesseinrichtung ist. Abhängig von der Position x des Flüssigkeitsmeniskus 20 bezüglich des Kanals 12 wird durch die Detektionseinrichtung 32 eine Kapazität C(x) gemessen.

An dieser Stelle sei angemerkt, daß anstatt der kapazitiven Detektionseinrichtung beispielsweise auch eine optische Detektionseinrichtung verwendet werden kann, welche angeordnet ist, um in einem Erfassungsdurchgang den Kanal abzutasten, wobei der Flüssigkeitsmeniskus dann festgestellt wird, wenn sich entweder ein durch den Kanal transmittiertes Licht oder ein vom Kanalboden reflektiertes Licht von einem hohen Pegel zu einem niedrigen Pegel verändert. Im Falle einer optisch arbeitenden Detektionseinrichtung ist es jedoch erforderlich, daß zumindest der Deckel oder der Boden des Kanals 12 für die Wellenlänge des verwendeten Lichts zumindest teilweise transparent ist. Ein geeignetes Material für den Basiskörper ist beispielsweise das optisch transparente Material Polykarbonat, das vorteilhafterweise spritzgußmäßig verarbeitet werden kann.

Andere Erfassungsmethoden, wie z.B. induktive Verfahren, können ebenfalls verwendet werden, solange die Meßgröße durch Eigenschaften des gefüllten Abschnitts 12b des Kanals im Vergleich zu Eigenschaften des ungefüllten Abschnitts 12a des Kanals beeinflußt wird.

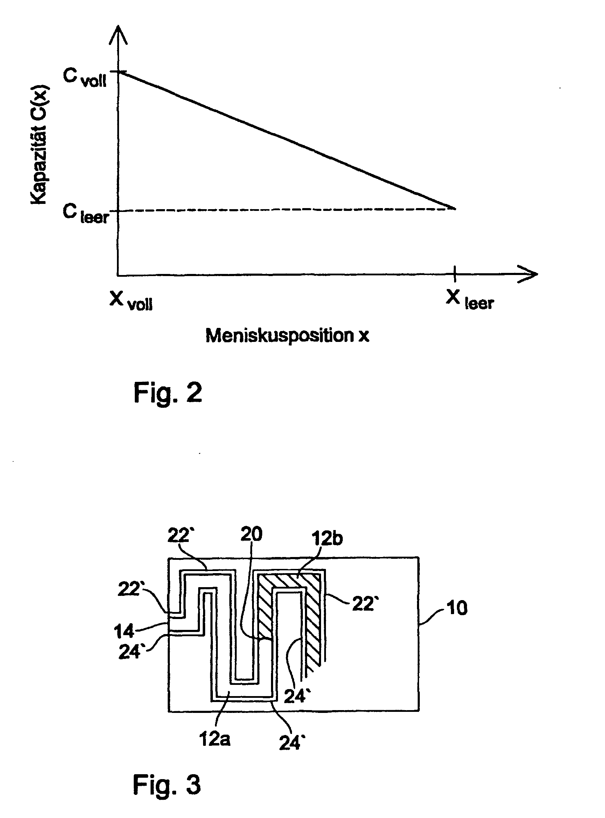

In Fig. 2 ist ein prinzipieller Kapazitätsverlauf der Kapazität C(x) über der Meniskusposition x dargestellt. Die Marke xᵥₒₗₗ bezeichnet den Fall, bei dem die bestimmungsgemäße maximale Füllmenge in das erfindungsgemäße Flüssigkeitsreservoir eingefüllt ist. Wie es weiterhin ausgeführt werden wird, ist dies nicht notwendigerweise die maximale Füllmenge. Stattdessen wird es bevorzugt, daß immer noch ein ungefüllter Abschnitt 12a des Kanals verbleibt, um die Verdunstung der Flüssigkeit zu minimieren. Dies bedeutet, daß sich der Flüssigkeitsmeniskus 20 auch bei vollgefülltem Kanal nicht notwendigerweise bis zum Auslaß 14 hin erstreckt. Die Marke xₗₑₑᵣ zeigt die Position des Flüssigkeitsmeniskus an, wenn das erfindungsgemäße Flüssigkeitsreservoir bestimmungsgemäß entleert ist. Nicht notwendigerweise muß sich der Flüssigkeitsmeniskus 20 jedoch unmittelbar am Auslaß 16 befinden, wenn noch Restflüssigkeit im Flüssigkeitsreservoir verbleiben soll. In dem Fall einer Dielektrizitätskonstante der Flüssigkeit 18 im gefüllten Abschnitt 12b, welche größer als die Dielektrizitätskonstante von dem Medium im ungefüllten Abschnitt 12a ist, welches normalerweise Luft sein wird, ist die Kapazität des erfindungsgemäßen Flüssigkeitsreservoirs am größten, wenn das Flüssigkeitsreservoir seinen bestimmungsgemäßen maximalen Füllstand hat. Dieser Kapazitätswert ist mit cᵥₒₗₗ in Fig. 2 bezeichnet. Im entgegengesetzten Fall, bei dem das erfindungsgemäße Flüssigkeitsreservoir maximal entleert ist, wird die dann noch vorhandene Kapazität Cₗₑₑᵣ genannt. Um im Sinne einer möglichst guten Meßgenauigkeit eine hohe Empfindlichkeit zu erreichen, besteht das Bestreben dahingehend, daß Cᵥₒₗₗ maximiert wird, während Cₗₑₑᵣ minimiert wird. Darüber hinaus wird zum Erreichen eines möglichst großen Füllvolumens je nach Anwendungsform die Mäanderlänge maximiert. Hierzu ist es nötig, daß die Isolationsschichten 28, 30 (Fig. 1), möglichst dünn gewählt werden, während die Mäanderfläche möglichst groß wird.

Daraus ist zu sehen, daß sich die einerseits bestehende Forderung nach einem von der Lage unabhängigen stabilen Flüssigkeitsmeniskus und die andererseits bestehende Forderung nach möglichst großer Mäanderfläche für eine hohe kapazitive Empfindlichkeit optimal ergänzen. Daher wird eine kapazitiv arbeitende Detektionseinrichtung für das erfindungsgemäße Flüssigkeitsreservoir bevorzugt.

Nachteilig an dem in Fig. 1 beschriebenen Grundkonzept ist die Tatsache, daß sowohl die Bodenelektrode 22 als auch die Deckelelektrode 24 optisch nicht transparent sind. Eventuell im gefüllten Abschnitt 12b des Kanals vorhandene Gasblasen sind nicht erfaßbar.

Zur Abhilfe dieser Eigenschaft können entweder die Deckelelektrode oder die Bodenelektrode oder aber beide Elektroden als Gitterelektrode ausgeführt sein, so daß die Füllqualität des Reservoirs entweder manuell oder automatisch beurteilt werden kann.

Alternativ können, wie es in Fig. 3 gezeigt ist, statt der im wesentlichen parallel zum Kanal verlaufenden Elektroden eine Anordnung von Vertikalelektroden 22', 24' verwendet werden, derart, daß die Elektroden nicht auf dem Basiskörper 10 wie in dem Fig. 1 gezeigten Ausführungsbeispiel aufgebracht sind, sondern vertikal zu den Hauptoberflächen des Basiskörpers. Die Elektroden 22', 24' folgen gewissermaßen dem Kanal 12 auf beiden Seiten desselben. In diesem Fall ist es, wenn der Deckel des Kanals oder der Boden des Kanals oder beide, so Deckel und Boden, optisch transparent ausgeführt werden, ohne weiteres möglich, in dem gefüllten Abschnitt 12b des Kanals vorhandene Gasblasen zu erkennen bzw. zu lokalisieren.

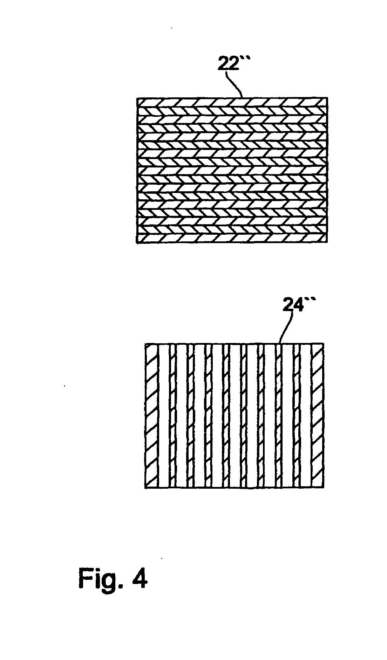

In Fig. 4 ist eine weitere Variante der Deckelelektrode 22" sowie der Bodenelektrode 24" gezeigt. Hier sind die beiden Elektroden 22" und 24" nicht mehr als durchgängige Fläche ausgeführt, sondern als einzelne voneinander isolierte Elektrodenstreifen, derart, daß sich zwischen einem Elektrodenstreifen der Bodenelektrode und einem Elektrodenstreifen der Deckelelektrode eine Einzelkapazität bildet. Durch paarweises Abfragen von mehreren Elektroden auf der Ober- oder Unterseite können ebenfalls Gasblasen innerhalb des gefüllten Abschnitts 12b des Kanals erkannt bzw. lokalisiert werden.

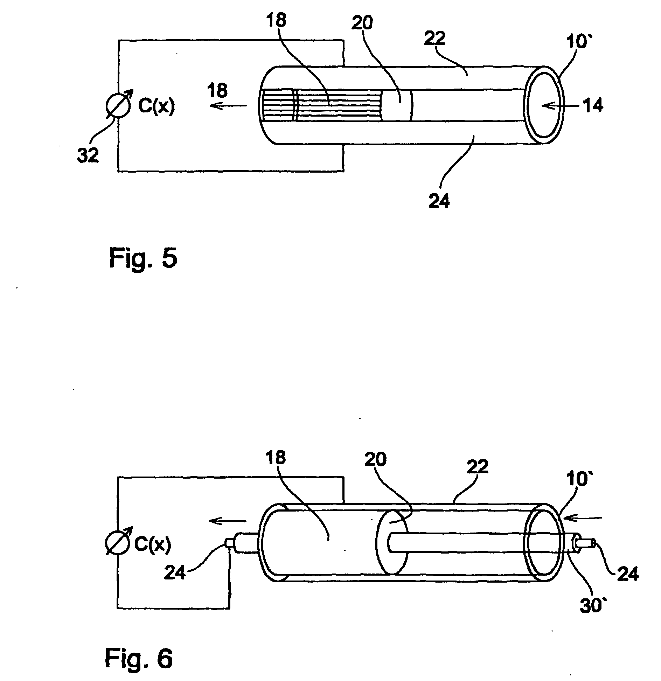

Fig. 5 zeigt eine alternative Ausführungsform des erfindungsgemäßen Flüssigkeitssensors, bei dem der Basiskörper 10' als Schlauch ausgeführt ist. Auf der Oberseite bzw. auf der Unterseite des Schlauches sind jeweils eine Elektrode 22 bzw. 24 aufgebracht, welche ebenso wie bei dem in Fig. 1 gezeigten Ausführungsbeispiel kontaktiert und mit einer kapazitiven Detektionseinrichtung versehen sind, um die Position des Meniskus 20 entlang der Länge des Schlauchs 10' zu bestimmen. Es sei darauf hingewiesen, daß bei dem in Fig. 5 gezeigten Ausführungsbeispiel keine extra vorhandenen Isolationsschichten benötigt werden, da die Schlauchwandung selbst als Isolationsschicht wirkt, um die Flüssigkeit 18 und die beiden Elektroden 22, 24 voneinander galvanisch zu entkoppeln.

Fig. 6 zeigt eine weitere Ausführungsform des erfindungsgemäßen Flüssigkeitsreservoirs, bei dem der Basiskörper ebenfalls als Schlauch 10' ausgeführt ist. Im Gegensatz zu dem in Fig. 5 gezeigten Ausführungsbeispiel wird bei dem in Fig. 6 gezeigten Ausführungsbeispiel eine gewissermaßen koaxiale Elektrodenanordnung verwendet. Die äußere Oberfläche des Schlauches 10' ist dabei vollständig von der einen Elektrode 22 umgeben, während die andere Elektrode 24 durch einen in dem Schlauch befindlichen Leiter 24 gebildet ist, welcher durch eine Isolationsschicht 30' von der Flüssigkeit 18 galvanisch entkoppelt ist. Die Elektrode 24 kann entweder lose im Schlauch liegen, oder aber konzentrisch in der Mitte fixiert werden. Liegt dieselbe lose im Schlauch, so ist es jedoch erforderlich, daß das Medikamentenreservoir in Schlauchform während einer Dosierung ruhig gehalten wird. Wird seine Position geändert, so muß dasselbe erneut kalibriert werden, um eventuelle Änderungen zwischen der isolierten inneren Elektrode 24 bezüglich der auf dem Schlauchmantel angeordneten äußeren Elektrode 22 zu berücksichtigen.

Im nachfolgenden wird auf Dimensionierungskriterien des in Fig. 1 gezeigten erfindungsgemäßen Flüssigkeitsreservoirs mit einem mäanderförmig gefalteten Kanal eingegangen. Wie es bereits bezugnehmend auf Fig. 2 ausgeführt worden ist, besteht das Bestreben, die Leerkapazität Cₗₑₑᵣ zu minimieren, und die Vollkapazität Cᵥₒₗₗ zu maximieren. Die Leer-Kapazität ergibt sich aus einer Parallelschaltung einer Kanalkapazität des mit Luft gefüllten Kanals und einer sogenannten Basiskörperkapazität, die durch den Rahmen des Basiskörpers und die Stege 26 (Fig. 1) des Basiskörpers gebildet wird. Aus einer Betrachtung ergibt sich, daß für eine möglichst kleine Leerkapazität ein Material für den Basiskörper gewählt werden sollte, das eine geringe Dielektrizitätskonstante aufweist. Desweiteren wird es bevorzugt, die Fläche der Stege und des Rahmens möglichst klein zu halten, bzw. die Elektrode möglichst lediglich auf dem Kanal aufzubringen, jedoch nicht zu stark über einen Bereich des Basiskörpers, der nicht vom Kanal besetzt ist.

Die Nutz-Kapazität ergibt sich aus der Differenz zwischen cᵥₒₗₗ und cₗₑₑᵣ. Sie sollte möglichst groß sein. Dies wird dadurch erreicht, daß die Mäanderfläche im Vergleich zur durch die Stege und den Rahmen eingenommenen Fläche möglichst groß gemacht wird. Darüber hinaus sollten, wie es bereits ausgeführt worden ist, sowohl der Kanaldeckel als auch der Kanalboden möglichst dünn gehalten werden.

Zusammenfassend wird festgestellt, daß das erfindungsgemäße Flüssigkeitsreservoir mit Füllstandsmessung so gestaltet ist, daß bei einer Entleerung des Reservoirs der Flüssigkeitsmeniskus, welcher die Grenzfläche zwischen Flüssigkeit und Luft darstellt, definiert geführt wird, so daß durch geeignete Detektionsverfahren der Füllstand des Reservoirs gemessen wird. Die Gefäßwände des Reservoirs werden bevorzugt so gestaltet, daß der Durchmesser des Reservoirs klein ist gegenüber der Länge des Reservoirs. Dies kann einerseits durch einen mäanderförmig gefalteten Kanal oder andererseits durch einen langen Schlauch erreicht werden.

Eine Störgröße für die Füllstandsmessung bzw. für den Betrieb von Flüssigkeitsreservoiren überhaupt ist die Verdunstung der Flüssigkeit. Verdunsten kann die Flüssigkeit vor allem am Meniskus. Bei einer Verdunstung treten Flüssigkeitsmoleküle in die Gasphase über. Dies führt effektiv- zu einer kleinen unerwünschten Bewegung des Meniskus, obwohl nichts dosiert wird. Die Verdunstungsrate, d.h. die Medikamentenmenge, die pro Zeit in die Gasphase übertritt, hängt neben der Sättigung der Luft mit Medikamentenmolekülen vor allem auch von der Größe der freien Oberfläche zwischen Medikament und Luft ab. Je kleiner daher der Durchmesser des Kanals ist, desto geringer ist die störende Verdunstung.

Die Verdunstung kann vor allem langfristig störend sein, beispielsweise wenn das gefüllte Reservoir über einen längeren Zeitraum gelagert wird. Um hier die Verdunstung weiter zu reduzieren, können geeignete Maßnahmen getroffen werden.

Eine Maßnahme besteht darin, das Flüssigkeitsreservoir nicht vollständig zu füllen, so daß eine geeignet lange Luftstrecke verbleibt. Es wird sich ein Konzentrationsgradient an in die Gasphase übergetretenen Flüssigkeitsmolekülen längs dieses ungefüllten Abschnitts des Kanals ausbilden. Je länger diese Diffusionsstrecke für in die Gasphase übergetretene Flüssigkeitsmoleküle ist, desto geringer ist auch die sich einstellende Verdunstung.

Eine andere Maßnahme besteht darin, die Luftstrecke an einer Stelle signifikant zu verringern, beispielsweise auf 0,05 mm. Dadurch wird der Austausch an Molekülen zwischen der Flüssigkeit in dem gefüllten Abschnitt des Kanals und dem Gas in dem ungefüllten Abschnitt des Kanals weiter verringert.

Eine weitere Maßnahme besteht darin, die luftseitige Einlaßöffnung mit einer semipermeablen Membran zu versiegeln, welche nur für Luftmoleküle, nicht aber für Medikamentenmoleküle durchlässig ist, derart, daß sich eine rasche Sättigung der eingeschlossenen Luft mit dem Medikament einstellt. Diese Versiegelung kann besonders für Flüssigkeitsreservoire mit Medikamenten den weiteren Vorteil eines keimfreien Abschlusses mit sich bringen.

Zusammenfassend kann daher festgestellt werden, daß die erfindungsgemäßen Vorteile umso signifikanter werden, je kleiner die Querschnittsdimensionierung des Kanals gewählt wird. Die konkreten Maße werden jedoch von der unmittelbar verwendeten Flüssigkeit und von dem Material abhängen, aus dem die Innenwand des Kanals gebildet ist.

Ein Anwendungsgebie des Flüssigkeitsreservoirs besteht in dem erfindungsgemäßen Dosiersystem, welches neben einem Flüssigkeitsreservoir eine Einrichtung zum Absaugen von in das Flüssigkeitsreservoir füllbarer Flüssigkeit, welche mit dem Auslaß verbunden ist, und/oder eine Einrichtung zum Pumpen von Gas in den ungefüllten Abschnitt, welche mit dem Einlaß verbunden ist, aufweist. Das erfindungsgemäße Dosiersystem umfaßt ferner eine Steuereinrichtung zum Steuern der Einrichtung zum Absaugen und/oder der Einrichtung zum Pumpen, wobei die Steuereinrichtung mit der Detektionseinrichtung des Flüssigkeitsreservoirs gekoppelt ist, um abhängig von der Position des Flüssigkeitsmeniskus eine vorbestimmte Menge an Flüssigkeit über den Auslaß des Kanals aus dem Kanal abzugeben.

Das Dosiersystem kann ferner in ein Entnahmesystem umfunktioniert werden. Dann ist die mit dem "Auslaß" verbundene Einrichtung als Pumpe ausgeführt, damit die z. B. aus dem Körper zu entnehmende Flüssigkeit über den Auslaß in das Flüssigkeitsreservoir befördert wird. Dies kann alternativ auch dadurch erreicht werden, daß die mit dem Einlaß verbundene Einrichtung als Sauger ausgeführt ist.

Das Dosiersystem und das Entnahmesystem können ferner erfindungsgemäß kombiniert werden, derart, daß im Falle nur einer vorhandenen Pumpe/Saugeinrichtung dieselbe beide Funktionen erfüllen kann, oder daß z. B. eine Pumpe nur fürs Dosieren am Einlaß angebracht ist, und eine Saugeinrichtung nur fürs Entnehmen mit dem Auslaß kommuniziert.

## Patentansprüche

1. Dosiersystem mit folgenden Merkmalen:
einem Flüssigkeitsreservoir mit Füllstandsmessung, mit folgenden Merkmalen:
einem Basiskörper (10; 10')
einem in dem Basiskö-rper ausgebildeten Kanal (12) mit einem Einlaß (14) und einem Auslaß (16), wobei die Querschnitts-Dimensionierung des Kanals (12) so gewählt ist, daß eine in den Kanal füllbare Flüssigkeit (18) einen Flüssigkeitsmeniskus (20) bildet, der einen mit Flüssigkeit gefüllten Abschnitt (12b) des Kanals von einem ungefüllten Abschnitt des Kanals (12a) abgrenzt, in dem keine Flüssigkeit vorhanden ist, wobei die Position des Flüssigkeitsmeniskus (20) bezüglich des Kanals (12) von der Ausrichtung des Flüssigkeitsreservoirs im wesentlichen unabhängig ist; und
einer Detektionseinrichtung (32, 22, 24; 22', 24'; 22", 24") zum Erfassen der Position des Flüssigkeitsmeniskus (20) bezüglich des Kanals, um aus der Position des Flüssigkeitsmeniskus den Füllstand und/oder eine Änderung des Füllstands des Flüssigkeitsreservoirs zu erhalten;
einer Einrichtung zum Absaugen einer in das Flüssigkeitsreservoir füllbaren Flüssigkeit, wobei die Einrichtung zum Absaugen mit dem Auslaß (16) verbunden ist, und/oder einer Einrichtung zum Pumpen von Gas in den ungefüllten Abschnitt des Kanals, wobei die Einrichtung zum Pumpen mit dem Einlaß verbunden ist; und
einer Steuereinrichtung zum Steuern der Einrichtung zum Absaugen und/oder der Einrichtung zum Pumpen, wobei die Steuereinrichtung mit der Detektionseinrichtung (32) des Flüssigkeitsreservoirs gekoppelt ist, um abhängig von der Position des Flüssigkeitsmeniskus (20) eine vorbestimmte Menge an Flüssigkeit über den Auslaß (16) des Kanals aus dem Kanal (12) abzugeben,
**dadurch gekennzeichnet, daß** die Detektionseinrichtung (32) kapazitiv ist und zwei voneinander elektrisch isolierte Elektroden (22, 24; 22', 24'; 22", 24") aufweist, die an dem Basisköxper (10; 10') so angebracht sind, daß sich zwischen ihnen sowohl der gefüllte (12b) als auch der ungefüllte Abschnitt (12a) des Kanals erstreckt, wodurch ein zwischen den zwei Elektroden erzeugbares elektrisches Feld sowohl in dem gefüllten Abschnitt des Kanals, als auch in dem ungefüllten Abschnitt des Kanals vorhanden ist, und wodurch eine Positionsänderung des Flüssigkeitsmeniskus zu einer proportionalen Kapazitätsänderung führt.

2. Kombiniertes Dosier/Entnahmesystem mit folgenden Merkmalen:
einem Flüasigkeitsreservoir mit Füllstandsmessung, mit folgenden Merkmalen:
einem Basiskörper (10; 10')
einem in dem Basisközper ausgebildeten Kanal (12) mit einem Einlaß (14) und einem Auslaß (16), wobei die Querschnitts-Dimensionierung des Kanals (12) so gewählt ist, daß eine in den Kanal füllbare Flüssigkeit (18) einen Flüssigkeitsmeniskus (20) bildet, der einen mit Flüssigkeit gefüllten Abschnitt (12b) des Kanals von einem ungefüllten Abschnitt des Kanals (12a) abgrenzt, in dem keine Flüssigkeit vorhanden ist, wobei die Position des Flüssigkeitsmeniskus (20) bezüglich des Kanals (12) von der Ausrichtung des Flüssigkeitsreservoirs im wesentlichen unabhängig ist; und
einer Detektionseinrichtung (32, 22, 24; 22' , 24'; 22", 24") zum Erfassen der Position des Flüssigkeitsmeniskus (20) bezüglich des Kanals, um aus der Position des Flüssigkeitsmeniskus den Füllstand und/oder eine Änderung des Füllstands des Flüssigkeitsreservoirs zu erhalten;
einer ersten Einrichtung zum Pumpen einer Flüssigkeit in das Flüssigkeitsreservoir bzw. zum Absaugen einer Flüssigkeit aus dem Flüssigkeitsreservoir, wobei die erste Einrichtung zum Pumpen bzw. Absaugen mit dem Auslaß (16) verbunden ist, und/oder einer zweiten Einrichtung zum Absaugen von Gas aus dem ungefüllten Abschnitt des Kanals bzw. zum Pumpen von Gas in den ungefüllten Abschnitt des Kanals, wobei die zweite Einrichtung zum Absaugen bzw. Pumpen mit dem Einlaß verbunden ist; und
einer Steuereinrichtung zum Steuern der ersten Einrichtung und/oder der zweiten Einrichtung, wobei die Steuereinrichtung mit der Detektionseinrichtung (32) des Flüssigkeitsreservoirs gekoppelt ist, um abhängig von der Position des Flüssigkeitsmeniskus (20) eine vorbestimmte Menge an Flüssigkeit über den Auslaß (16) des Kanals in den Kanal (12) hinein zu befördern bzw. über den Auslaß des Kanals aus dem Kanal heraus abzugeben,
**dadurch gekennzeichnet, daß** die Detektionseinrichtung (32) kapazitiv ist und zwei voneinander elektrisch isolierte Elektroden (22, 24; 22', 24'; 22", 24") aufweist, die an dem Basiskörper (10; 10') so angebracht sind, daß sich zwischen ihnen sowohl der gefüllte (12b) als auch der ungefüllte Abschnitt (12a) des Kanals erstreckt, wodurch ein zwischen den zwei Elektroden erzeugbares elektrisches Feld sowohl in dem gefüllten Abschnitt des Kanals, als auch in dem ungefüllten Abschnitt des Kanals vorhanden ist, und wodurch eine positionsänderung des Flüssigkeitsmeniskus zu einer proportionalen Kapazitätsänderung führt.

3. Vorrichtung nach Anspruch 1 oder 2, bei der der Kanal (12) mäanderförmig gefaltet ist, um einen zu äußeren Abmessungen des Basiskörpers (10) vergleichsweise langen Kanal zu erhalten.

4. Vorrichtung nach Anspruch 1 oder 2, bei der der Basiskörper 10' ein Schlauch ist, und der Kanal durch die Schlauchöffnung gebildet ist.

5. Vorrichtung nach Anspruch 1 oder 2, bei der der Kanal (12) eine elliptische und vorzugsweise kreisförmige Querschnittsdimensionierung aufweist, wobei die kleine Achse des elliptischen Querschnitts bzw. der Durchmesser des kreisförmigen Querschnitts so klein gewählt ist, daß die Position des Meniskus (20) im wesentlichen durch die Oberflächenspannung der Flüssigkeit (18) sowie durch die Grenzflächenspannung zwischen der Flüssigkeit (18) und dem Material der Kanalwand bestimmt wird, während die Schwerkraft in wesentlichen keinen Einfluß auf die Position des Meniskus (20) hat.

6. Vorrichtung nach Anspruch 1 oder 2, bei der der Kanal (12) eine rechteckige Querschnitts-Dimensionierung hat, wobei die kleine Seite der rechteckigen Querschnitts-Dimensionierung so klein gewählt ist, daß die Position des Meniskus (20) im wesentlichen durch die Oberflächenspannung der Flüssigkeit (18) sowie durch die Grenzflächenspannung zwischen der Flüssigkeit (18) und dem Material der Kanalwand bestimmt wird, während die Schwerkraft in wesentlichen keinen Einfluß auf die Position des Meniskus (20) hat.

7. Vorrichtung nach Anspruch 1 oder 2, bei der die Länge des Kanals so gewählt ist, daß bei bestimmungsgemäß gefülltem Flüssigkeitsreservoir der Abschnitt des Kanals, in dem keine Flüssigkeit vorhanden ist, eine Mindestlänge hat, die durch eine zulässige Verdunstungsrate für die Flüssigkeit festgelegt ist.

8. Vorrichtung nach Anspruch 1 oder 2, bei der in dem Abschnitt des Kanals, in dem bei bestimmungsgemäß gefülltem Flüssigkeitsreservoir keine Flüssigkeit vorhanden ist, eine Einschnürung vorhanden ist, deren Querschnitts-Dimensionierung durch die zulässige Verdunstungsrate beeinflußt wird.

9. Vorrichtung nach Anspruch 1 oder 2, bei der an dem Einlaß eine versiegelung mittels einer semipermeablen Membran vorhanden ist, die für Flüssigkeitsmoleküle undurchlässig ist.

10. Vorrichtung nach Anspruch 1 oder 2, bei der der Basiskörper (10') ein Schlauch ist, und bei der beide Elektroden (22, 24) auf der Außenoberfläche des Schlauches angeordnet sind und sich entlang der Länge des Schlauches erstrecken.

11. Vorrichtung nach Anspruch 1 oder 2, bei der der Basiskörper (10') ein Schlauch ist, und bei der eine Elektrode (22) auf der Außenoberfläche des Schlauches angeordnet ist, und die andere Elektrode (24) innerhalb des Schlauches positioniert ist und durch eine Isolatiansschicht (30') von der Flüssigkeit (18) galvanisch getrennt ist.

12. Vorrichtung nach Anspruch 1 oder 2,
bei der eine Elektrode (24) als Bodenelektrode ausgeführt ist, und bei der die andere Elektrode (22) als Deckelelektrode ausgeführt ist, wobei der in dem Basiskörper (10) vorhandene Kanal durch die Boden- und die Deckelelektrode direkt begrenzt ist, oder durch eine elektrisch isolierende Schicht (30) zwischen der Bodenelektrode (24) und dem Kanal (12) und durch eine elektrisch isolierende Schicht (28) zwischen der Deckelelektrode (22) und dem Kanal (12) begrenzt ist, wobei die elektrisch isolierenden Schichten einstückig mit dem Basiskörper und/oder mit der Boden- und der Deckelelektrode ausgeführt sind.

13. Vorrichtung nach Anspruch 12, bei der die Deckelelektrode, die Bodenelektrode oder beide als Gitterelektroden ausgeführt sind, um Gasblasen in den gefüllten Abschnitt des Kanals optisch detektieren zu können.

14. Vorrichtung nach Anspruch 12, bei der die Deckelelektrode (22") und/oder die Bodenelektrode (24") aus einer Mehrzahl von Einzelelektroden bestehen, so daß zwischen der Bodenelektrode und der Deckelelektrode eine Mehrzahl von Einzelkapazitäten gebildet sind, wobei die Einzelkapazitäten unabhängig voneinander bestimmbar sind, um Gasblasen in dem gefüllten Abschnitt kapazitiv zu detektieren.

15. Vorrichtung nach Anspruch 1 oder 2, bei der die zwei Elektroden (22', 24') vertikal bezüglich von Hauptoberflächen des Basiskörpers (10) angeordnet sind und sich jeweils entlang des Kanals erstrecken.

16. Vorrichtung nach Anspruch 1 oder 2, bei der der Kanal auf zumindest einer Seite desselben optisch transparent ist, so daß eine Beurteilung der Füllqualität manuell oder automatisch beurteilbar ist.

## Claims

1. Dosing system, comprising:
a liquid reservoir with level measurement, comprising:
a base body (10; 10');
a channel (12) implemented in the base body and having an inlet (14) and an outlet (16), the dimensions of the channel cross-section (12) being selected such that a liquid (18) which may be filled into the channel forms a liquid meniscus (20) which demarcates a section (12b) of the channel filled with liquid from an unfilled section (12a) of the channel in which no liquid is present, the position of the liquid meniscus (20) in relation to the channel (12) being substantially independent of the orientation of the liquid reservoir; and
detection means (32, 22, 24; 22', 24'; 22", 24") for detecting the position of the liquid meniscus (20) in relation to the channel so as to obtain the level and/or a change in the level of the liquid reservoir from the position of the liquid meniscus;
means for exhausting a liquid which may be filled into the liquid reservoir, the means for exhausting being connected to the outlet (16), and/or means for pumping gas into the unfilled section of the channel, the means for pumping being connected to the inlet; and
control means for controlling the means for exhausting and/or the means for pumping, the control means being coupled to the detection means (32) of the liquid reservoir so as to dispense a predetermined amount of liquid from the channel (12) via the outlet (16) of the channel, depending on the position of the liquid meniscus (20);
**characterized in that** the detection means (32) are capacitive and comprise two electrodes (22, 24; 22', 24'; 22", 24") electrically insulated from each other and mounted on the base body (10; 10') such that both the filled (12b) and the unfilled section (12a) of the channel extend between same, whereby an electric field that may be generated between the two electrodes is present both in the filled section of the channel and in the unfilled section of the channel, and whereby a change in the position of the liquid meniscus leads to a proportional change in capacitance.

2. Combined dosing/withdrawal system, comprising:
a liquid reservoir with level measurement, comprising:
a base body (10; 10');
a channel (12) implemented in the base body and having an inlet (14) and an outlet (16), the dimensions of the channel cross-section (12) being selected such that a liquid (18) which may be filled into the channel forms a liquid meniscus (20) which demarcates a section (12b) of the channel filled with liquid from an unfilled section (12a) of the channel in which no liquid is present, the position of the liquid meniscus (20) in relation to the channel (12) being substantially independent of the orientation of the liquid reservoir; and
detection means (32, 22, 24; 22', 24'; 22", 24") for detecting the position of the liquid meniscus (20) in relation to the channel so as to obtain the level and/or a change in the level of the liquid reservoir from the position of the liquid meniscus;
first means for pumping a liquid into the liquid reservoir and/or for exhausting a liquid from the liquid reservoir, the first means for pumping and/or exhausting being connected to the outlet (16), and/or second means for exhausting gas from the unfilled section of the channel and/or for pumping gas into the unfilled section of the channel, the second means for exhausting and/or pumping being connected to the inlet; and
control means for controlling the first means and/or the second means, the control means being coupled to the detection means (32) of the liquid reservoir so as to convey a predetermined amount of liquid into the channel (12) via the outlet (16) of the channel and/or dispense a predetermined amount of liquid from the channel via the outlet of the channel, depending on the position of the liquid meniscus (20),
**characterized in that** the detection means (32) are capacitive and comprise two electrodes (22, 24; 22', 24'; 22", 24") electrically insulated from each other and mounted on the base body (10; 10') such that both the filled (12b) and the unfilled section (12a) of the channel extend between same, whereby an electric field that may be generated between the two electrodes is present both in the filled section of the channel and in the unfilled section of the channel, and whereby a change in the position of the liquid meniscus leads to a proportional change in capacitance.

3. Apparatus as claimed in claim 1 or 2, wherein the channel (12) is folded in a meander-like fashion so as to obtain a comparatively long channel in relation to outer dimensions of the base body (10).

4. Apparatus as claimed in claim 1 or 2, wherein the base body (10') is a hose, and the channel is formed by the opening of the hose.

5. Apparatus as claimed in claim 1 or 2, wherein the channel (12) has elliptical and preferably circular cross-sectional dimensions, the small axis of the elliptical cross-section and/or the diameter of the circular cross-section being selected to be so small that the position of the meniscus (20) is determined substantially by the surface tension of the liquid (18) as well as by the interfacial tension between the liquid (18) and the material of the channel wall, whereas gravity has substantially no influence on the position of the meniscus (20).

6. Apparatus as claimed in claim 1 or 2, wherein the channel (12) has rectangular cross-sectional dimensions, the small side of the rectangular cross-sectional dimensions being selected so small that the position of the meniscus (20) is determined substantially by the surface tension of the liquid (18) as well as by the interfacial tension between the liquid (18) and the material of the channel wall, whereas gravity has substantially no influence on the position of the meniscus (20).

7. Apparatus as claimed in claim 1 or 2, wherein the length of the channel is selected such that, with the liquid reservoir filled as directed, the section of the channel in which no liquid is present has a minimum length determined by an admissible evaporation rate for the liquid.

8. Apparatus as claimed in claim 1 or 2, wherein in the section of the channel in which no liquid is present, with the liquid reservoir filled as directed, a constriction is present, the cross-sectional dimensioning of which is influenced by the admissible evaporation rate.

9. Apparatus as claimed in claim 1 or 2, wherein a sealing in the form of a semi-permeable membrane which is impermeable for liquid molecules is present at the inlet.

10. Apparatus as claimed in claim 1 or 2, wherein the base body (10') is a hose, and wherein both electrodes (22, 24) are arranged on the outer surface of the hose and extend along the length of the hose.

11. Apparatus as claimed in claim 1 or 2, wherein the base body (10') is a hose, and wherein one electrode (22) is arranged on the outer surface of the hose, and the other electrode (24) is positioned within the hose and is separated from the liquid (18) by an insulating layer (30') by means of conductive separation.

12. Apparatus as claimed in claim 1 or 2,
wherein one electrode (24) is implemented as a bottom electrode, and wherein the other electrode (22) is implemented as a cap electrode, the channel present in the base body (10) being directly bounded by the bottom and cap electrodes or being bounded by an electrically insulating layer (30) between the bottom electrode (24) and the channel (12) and by an electrically insulating layer (28) between the cap electrode (22) and the channel (12), the electrically insulating layers being implemented integrally with the base body and/or the bottom and cap electrodes.

13. Apparatus as claimed in claim 12, wherein the cap electrode, the bottom electrode or both are implemented as grid electrodes so as to be able to optically detect any gas bubbles in the filled section of the channel.

14. Apparatus as claimed in claim 12, wherein the cap electrode (22") and/or the bottom electrode (24") consist of a plurality of individual electrodes, so that a plurality of individual capacitances are formed between the bottom electrode and the cap electrode, the individual capacitances being determinable independently of each other so as to capacitively detect any gas bubbles in the filled section.

15. Apparatus as claimed in claim 1 or 2, wherein both electrodes (22', 24') are arranged vertically in relation to main surfaces of the base body (10) and each extend along the channel.

16. Apparatus as claimed in claim 1 or 2, wherein the channel is optically transparent on at least one side of same, so that an evaluation of the filling quality may be performed manually or automatically.

## Revendications

1. Système de dosage aux caractéristiques suivantes :
un réservoir à liquide avec mesure du niveau de liquide, aux caractéristiques suivantes :
un corps de base (10 ; 10')
un canal réalisé dans le corps de base (12) avec une entrée (14) et une sortie (16), le dimensionnement de section du canal (12) étant choisi de sorte qu'un liquide (18) pouvant être chargé dans le canal forme un ménisque de liquide (20) délimitant un segment du canal (12b) rempli de liquide d'un segment du canal non rempli (12a) dans lequel n'est pas présent de liquide, la position du ménisque de liquide (20) par rapport au canal (12) étant substantiellement indépendante de l'orientation du. réservoir à liquide ; et
un dispositif de détection (32, 22, 24 ; 22', 24' ; 22", 24") destiné à détecter la position du ménisque de liquide (20) par rapport au canal, pour obtenir à partir de la position du ménisque de liquide le niveau de remplissage et/ou une variation du niveau de remplissage du réservoir à liquide ;
un dispositif destiné à aspirer un liquide pouvant être chargé dans le réservoir à liquide, le dispositif destiné à aspirer étant relié à la sortie (16), et/ou un dispositif destiné à pomper du gaz dans le segment non rempli du canal, le dispositif de pompage étant relié à l'entrée ; et
un dispositif de commande du dispositif destiné à aspirer et/ou du dispositif de pompage, le dispositif de commande étant couplé au dispositif de détection (32) du réservoir à liquide, pour délivrer du canal (12), en fonction de la position du ménisque de liquide (20), une quantité prédéterminée de liquide, par l'intermédiaire de la sortie (16) du canal,
**caractérisé par le fait que** le dispositif de détection (32) est capacitif et présente deux électrodes (22, 24 ; 22', 24' ; 22", 24") isolées électriquement l'une de l'autre qui sont placés sur le corps de base (10 ; 10') de sorte que s'étendent entre elles tant le segment rempli (12b) que le segment non rempli (12a) du canal, d'où un champ électrique pouvant être généré entre les deux électrodes est présent tant dans le segment rempli du canal que dans le segment non rempli du canal, et d'où une variation de position du ménisque de liquide entraîne une variation de capacité proportionnelle.

2. Système combiné de dosage/prélèvement aux caractéristiques suivantes :
un réservoir liquide avec la mesure du niveau liquide, avec les caractéristiques suivantes :
un corps de base (10 ; 10')
un canal (12) réalisé dans le corps de base avec une entrée (14) et une sortie (16), le dimensionnement de section du canal (12) étant choisi de sorte qu'un liquide (18) pouvant être chargé dans le canal forme un ménisque de liquide (20) qui délimite un segment (12b) du canal rempli de liquide d'un segment non rempli du canal (12a) dans lequel n'est pas présent de liquide, la position du ménisque de liquide (20) par rapport au canal (12) étant sensiblement indépendante de l'orientation du réservoir à liquide ; et
un dispositif de détection (32, 22, 24 ; 22', 24' ; 22", 24") destiné à détecter la position du ménisque de liquide (20) par rapport au canal, pour obtenir à partir de la position du ménisque de liquide le niveau de remplissage et/ ou une variation du niveau de remplissage du réservoir à liquide ;
un premier dispositif destiné à pomper un liquide dans le réservoir à liquide ou à aspirer un liquide du réservoir à liquide, le premier dispositif destiné à pomper ou aspirer étant relié à la sortie (16), et/ou un deuxième dispositif destiné à aspirer du gaz du segment non rempli du canal ou à pomper du gaz dans le segment non rempli du canal, le deuxième dispositif destiné à pomper ou aspirer étant relié à l'entrée ; et
un dispositif de commande destiné à commander le premier dispositif et/ou le deuxième dispositif, le dispositif de commande étant couplé au dispositif de détection (32) du réservoir à liquide, pour transporter, en fonction de la position du ménisque de liquide (20), une quantité prédéterminée de liquide, par l'intermédiaire de la sortie (16) du canal, vers le canal (12) ou pour la délivrer du canal par l'intermédiaire de la sortie,
**caractérisé par le fait que** le dispositif de détection (32) est capacitif et présente deux électrodes (22, 24 ; 22', 24'; 22", 24") isolées électriquement l'une de l'autre qui sont disposées sur le corps de base (10 ; 10') de sorte que s'étendent entre elles tant le segment rempli (12b) que le segment non rempli (12a) du canal, d'où un champ électrique pouvant être généré entre les deux électrodes est présent tant dans le segment rempli du canal que dans le segment non rempli du canal, et d'où une variation de position du ménisque de liquide entraîne une variation de capacité proportionnelle.

3. Dispositif selon la revendication 1 ou 2, dans lequel le canal (12) est replié en méandre, pour obtenir un canal comparativement long par rapport aux dimensions extérieures du corps de base (10).

4. Dispositif selon la revendication 1 ou 2, dans lequel le corps de base 10' est un flexible et le canal est formé par l'ouverture de flexible.

5. Dispositif selon la revendication 1 ou 2, dans lequel le canal (12) présente un dimensionnement de section elliptique et, de préférence, circulaire, le petit axe de la section elliptique ou le diamètre de la section circulaire étant choisi aussi réduit que la position du ménisque (20) soit substantiellement déterminé par la tension superficielle du liquide (18) ainsi que par la tension superficielle entre le liquide (18) et le matériau de la paroi de canal, tandis que la gravité n'a substantiellement pas d'influence sur la position du ménisque (20).

6. Dispositif selon la revendication 1 ou 2, dans lequel le canal (12) présente un dimensionnement de section rectangulaire, le petit côté du dimensionnement de section rectangulaire étant choisi aussi réduit que la position du ménisque (20) soit déterminé substantiellement par la tension superficielle du liquide (18) ainsi que par la tension superficielle entre le liquide (18) et le matériau de la paroi de canal, tandis que la gravité n'a substantiellement pas d'influence sur la position du ménisque (30).

7. Dispositif selon la revendication 1 ou 2, dans lequel la longueur du canal est choisie de sorte que, en cas de réservoir à liquide rempli de manière conventionnelle, le segment du canal dans lequel n'est pas présent de liquide ait une longueur minimale qui est fixée par un taux d'évaporation admissible du liquide.

8. Dispositif selon la revendication 1 ou 2, dans lequel est présente, dans le segment du canal dans lequel n'est pas présent de liquide en cas de réservoir à liquide rempli de manière conventionnelle, une constriction dont le dimensionnement de section est influencé par le taux d'évaporation admissible.

9. Dispositif selon la revendication 1 ou 2, dans lequel est présente, à l'entrée, une obturation étanche au moyen d'une membrane semi-perméable qui est imperméable aux molécules de liquide.

10. Dispositif selon la revendication 1 ou 2, dans lequel le corps de base (10') est un flexible et dans lequel les deux électrodes (22, 24) sont disposées sur la surface extérieure du flexible et s'étendent sur la longueur du flexible.

11. Dispositif selon la revendication 1 ou 2, dans lequel le corps de base (10') est un flexible et dans lequel une électrode (22) est disposée sur la surface extérieure du flexible et l'autre électrode (24) est positionnée à l'intérieur du flexible et est séparée galvaniquement du liquide (18) par une couche d'isolation (30').

12. Dispositif selon la revendication 1 ou 2,
dans lequel l'une électrode (24) est réalisée sous forme d'électrode de fond et dans lequel l'autre électrode (22) est réalisée sous forme d'électrode de couvercle, le canal présent dans le corps de base (10) étant limité directement par l'électrode de fond et l'électrode de couvercle, ou est limité par une couche d'isolation électrique (30) entre l'électrode de fond (24) et le canal (12) et par une couche d'isolation électrique (28) entre l'électrode de couvercle (22) et le canal (12), par lequel, les couches d'isolation électrique étant réalisées d'une seule pièce avec le corps de base et/ou avec l'électrode de fond et l'électrode de couvercle.

13. Dispositif selon la revendication 12, dans lequel l'électrode de couvercle, l'électrode de fond ou les deux sont réalisées sous forme d'électrodes en grille, afin de pouvoir détecter optiquement des bulles de gaz dans le segment rempli du canal.

14. Dispositif selon la revendication 12, dans lequel l'électrode de couvercle (22") et/ou l'électrode de fond (24") se composent d'une pluralité d'électrodes individuelles, de sorte qu'entre l'électrode de fond et l'électrode de couvercle soit formées une pluralité de capacités individuelles, les capacités individuelles pouvant être déterminées indépendamment l'une de l'autre, afin de détecter capacitivement des bulles de gaz dans le segment rempli.

15. Dispositif selon la revendication 1 ou 2, dans lequel les deux électrodes (22', 24') sont disposées verticalement par rapport aux surfaces principales du corps de base (10) et s'étendent, chacune, le long du canal.

16. Dispositif selon la revendication 1 ou 2, dans lequel le canal est, au moins d'un côté de ce dernier, optiquement transparent, de sorte qu'une évaluation de la qualité de remplissage puisse être appréciée manuellement ou automatiquement.
